# EUROPEAN PATENT SPECIFICATION

(11) **EP 3 672 492 B1**
(45) Date of publication and mention of the grant of the patent: **22.10.2025**
(21) Application number: 18847971.1
(22) Date of filing: 12.04.2018
(51) Int. Cl.: A61B 8/00, A61B 8/08

(54) **ULTRASOUND DIAGNOSIS APPARATUS AND METHOD OF OPERATING THE SAME**
ULTRASCHALLDIAGNOSEVORRICHTUNG UND VERFAHREN ZUM BETRIEB DAVON
APPAREIL DE DIAGNOSTIC À ULTRASONS ET SON PROCÉDÉ DE FONCTIONNEMENT

(30) Priority: 25.08.2017 US 201762550124 P; 27.12.2017 KR 20170181452
(43) Date of publication of application: 01.07.2020
(60) Divisional application: 25174052.8
(73) Proprietor: Samsung Medison Co., Ltd., Hongcheon-gun, Gangwon-do 25108 (KR)
(72) Inventor: JIN, Gil-ju, Hongcheon-gun Gangwon-do 25108 (KR); JO, Jae-moon, Hongcheon-gun Gangwon-do 25108 (KR); KIM, Yu-ri, Hongcheon-gun Gangwon-do 25108 (KR); AHN, Mi-jeoung, Hongcheon-gun Gangwon-do 25108 (KR)
(74) Representative: Arnold & Siedsma
(86) International application number: PCT/KR2018/004276
(87) International publication number: WO 2019/039697

(56) References cited:
- EP-A2- 2 829 234
- WO-A1-2017/009735
- JP-A- 2008 000 406
- JP-B2- 5 519 949
- KR-A- 20150 027 010
- KR-B1- 101 625 661
- KR-B1- 101 733 731
- US-A1- 2010 168 576
- US-A1- 2012 101 389
- US-A1- 2016 199 028

## Description

### Technical Field

The present disclosure relates to ultrasound diagnosis apparatuses and methods of operating the same, and more particularly, to an ultrasound diagnosis apparatus including at least one wired ultrasound probe and a plurality of wireless ultrasound probes and a method of operating the ultrasound diagnosis apparatus.

### Background Art

Ultrasound systems transmit ultrasound signals generated by transducers of an ultrasound probe to an internal part of an object and receive information about echo signals reflected therefrom, thereby obtaining an image of the internal part of the object. In particular, ultrasound systems are used for medical purposes including observation of an internal area of an object, detection of foreign substances, diagnosis of damage to the object, and imaging of characteristics.

Wireless ultrasound probes connected to an ultrasound diagnosis apparatus by using wireless communication are nowadays being developed in order to improve the operability of an ultrasound probe by removing a communication cable for transmitting and receiving ultrasound image data between the ultrasound probe and the ultrasound diagnosis apparatus and eliminating the inconvenience caused by the communication cable. However, at the present time, an ultrasound diagnosis apparatus including a wireless ultrasound probe may contain only one wireless ultrasound probe, and only one wireless ultrasound probe may be connected to the ultrasound diagnosis apparatus at a time. Furthermore, in the case of an ultrasound diagnosis apparatus including both wired and wireless ultrasound probes, when a user stops using the wired ultrasound probe and attempts to use the wireless ultrasound probe, the user suffers the inconvenience of having to manually pair the wireless ultrasound probe to the ultrasound diagnosis apparatus and activate the paired wireless ultrasound probe.
In addition to or as an alternative for the above reflection of the prior art, further US-2012/101389 is acknowledged here as a disclosure of prior art, relative to which at least features in characterizing portions of the appended independent claims are novel and impart an inventive step to the present disclosure.

### Disclosure of Invention

### Solution to Problem

Provided are ultrasound diagnosis apparatuses and methods of operating the same, including features and steps defined in the appended independent apparatus and method claims, respectively.

Additional aspects will be set forth in part in the description which follows and, in part, will be apparent from the description, or may be learned by practice of the presented embodiments.

### Advantageous Effects of Invention

The ultrasound diagnosis apparatus includes both at least one wired ultrasound probe and a plurality of wireless ultrasound probes, and is configured to detect an ultrasound probe being used by the user to examine the object among one of the at least one wired ultrasound probes and any of the plurality of wireless ultrasound probes, and activate the detected ultrasound probe directly without a separate pairing process, thereby increasing user convenience. In particular, even when an ultrasound probe being used is switched from a wired ultrasound probe to a wireless ultrasound probe or vice versa, the ultrasound diagnosis apparatus may automatically detect an ultrasound probe being used as a result of switching probes and activate the detected ultrasound probe to transmit ultrasound signals to the object and acquire ultrasound image data from the object.

### Brief Description of Drawings

These and/or other aspects will become apparent and more readily appreciated from the following description of the embodiments, taken in conjunction with the accompanying drawings in which:
FIG. 1 is a conceptual diagram illustrating an example in which an ultrasound diagnosis apparatus detects an ultrasound probe being used by a user, from among wired and wireless ultrasound probes, and activates the detected ultrasound probe, according to an embodiment;
FIG. 2 is a block diagram of a configuration of an ultrasound diagnosis apparatus according to an embodiment;
FIG. 3 is a flowchart of a method of operating an ultrasound diagnosis apparatus, according to the invention;
FIG. 4 is a flowchart of a method of operating an ultrasound diagnosis apparatus, according to another embodiment;
FIG. 5 is a block diagram of a configuration of an ultrasound diagnosis apparatus according to another embodiment;
FIG. 6 is a flowchart of a method of operating an ultrasound diagnosis apparatus, according to another embodiment;
FIG. 7 is a block diagram of a configuration of an ultrasound diagnosis apparatus including a wired ultrasound probe, according to an embodiment;
FIG. 8 is a block diagram of a configuration of an ultrasound diagnosis system including a wireless ultrasound probe, according to an embodiment; and
FIGS. 9A through 9C are diagrams illustrating ultrasound diagnosis apparatuses.

### Best Mode for Carrying out the Invention

In accordance with an aspect of the present disclosure, an ultrasound diagnosis apparatus includes features of the appended independent apparatus claim.

In accordance with another aspect of the present disclosure, a method involves steps defined in the appended independent method claim.

In accordance with another aspect of the present disclosure, a computer-readable recording medium having recorded thereon a computer program including instructions for executing operations to perform the above method.

### Mode for the Invention

Advantages and features of one or more embodiments of the present disclosure and methods of accomplishing the same may be understood more readily by reference to the following detailed description of the embodiments and the accompanying drawings. In this regard, the present embodiments may have different forms and should not be construed as being limited to the descriptions set forth herein. Rather, these embodiments are provided so that this disclosure will be thorough and complete and will fully convey the concept of the present embodiments to one of ordinary skill in the art, and the present disclosure will only be defined by the appended claims.

Terms used herein will now be briefly described and then one or more embodiments of the present disclosure will be described in detail.

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the disclosure. Thus, the terms used herein have to be defined based on the meaning of the terms together with the description throughout the specification.

When a part "includes" or "comprises" an element, unless there is a particular description contrary thereto, the part can further include other elements, not excluding the other elements. Also, the term "unit" in the embodiments of the present disclosure means a software component or hardware component such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

The buttons, trackballs, jog switches, and knobs included in the control panel 930 may be provided as a GUI to the main display 910 or the sub-display 920.

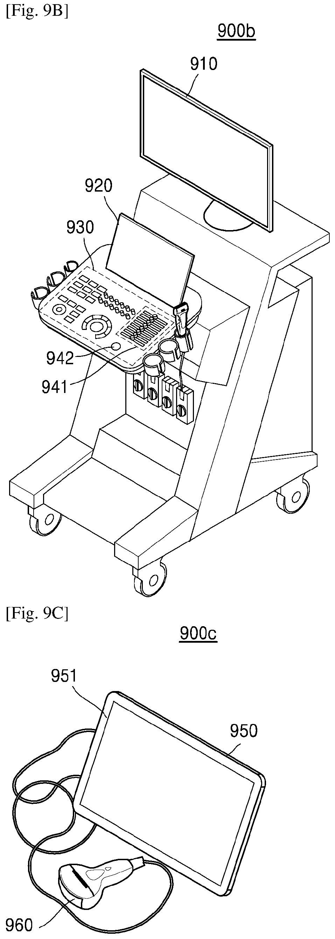

Referring to FIG. 9C, the ultrasound diagnosis apparatus 900c may include a portable device. An example of the portable ultrasound diagnosis apparatus 900c may include, for example, smart phones including probes and applications, laptop computers, personal digital assistants (PDAs), or tablet PCs, but an exemplary embodiment is not limited thereto.
The ultrasound diagnosis apparatus 900c may include the probe 960 and a main body 950. The probe 960 may be connected to one side of the main body 950 by wire or wirelessly. The main body 950 may include a touch screen 951. The touch screen 951 may display an ultrasound image, various pieces of information processed by the ultrasound diagnosis apparatus 900c, and a GUI.

The embodiments of the present disclosure can be written as computer programs and can be implemented in general-use digital computers that execute the programs using a computer-readable recording medium. The above-described embodiments of the present disclosure may be embodied in form of a computer-readable recording medium for storing computer executable command languages and data. The command languages may be stored in form of program codes and, when executed by a processor, may perform a certain operation by generating a certain program module. Also, when executed by a processor, the command languages may perform certain operations of the disclosed embodiments.

Examples of the computer-readable recording medium include magnetic storage media (e.g., ROM, floppy disks, hard disks, etc.), optical recording media (e.g., CD-ROMs or DVDs), etc.

While the present disclosure has been particularly shown and described with reference to exemplary embodiments thereof, it will be understood by those of ordinary skill in the art that various changes in form and details may be made therein without departing from the scope of the present disclosure as defined by the following claims. Accordingly, the above embodiments and all aspects thereof are examples only and are not limiting.

In the present specification, an "object" may be a human, an animal, or a part of a human or animal. For example, the object may be an organ (e.g., the liver, the heart, the womb, the brain, a breast, or the abdomen), a blood vessel, or a combination thereof. Furthermore, the "object" may be a phantom. The phantom means a material having a density, an effective atomic number, and a volume that are approximately the same as those of an organism. For example, the phantom may be a spherical phantom having properties similar to the human body.

Furthermore, in the present specification, a "user" may be, but is not limited to, a medical expert, such as a medical doctor, a nurse, a medical laboratory technologist, and a technician who repairs a medical apparatus.

Furthermore, in the present specification, the terms "first", "second", "1-1", etc. are only used to distinguish one component, element, object, image, pixel, or patch from another component, element, object, image, pixel, or patch. Thus, these terms are not limited to representing the order or priority among elements or components. Expressions such as "at least one of," when preceding a list of elements, modify the entire list of elements and do not modify the individual elements of the list.

Reference will now be made in detail to embodiments, examples of which are illustrated in the accompanying drawings. In this regard, the present embodiments may have different forms and should not be construed as being limited to the descriptions set forth herein. In the following description, well-known functions or constructions are not described in detail so as not to obscure the embodiments with unnecessary detail.

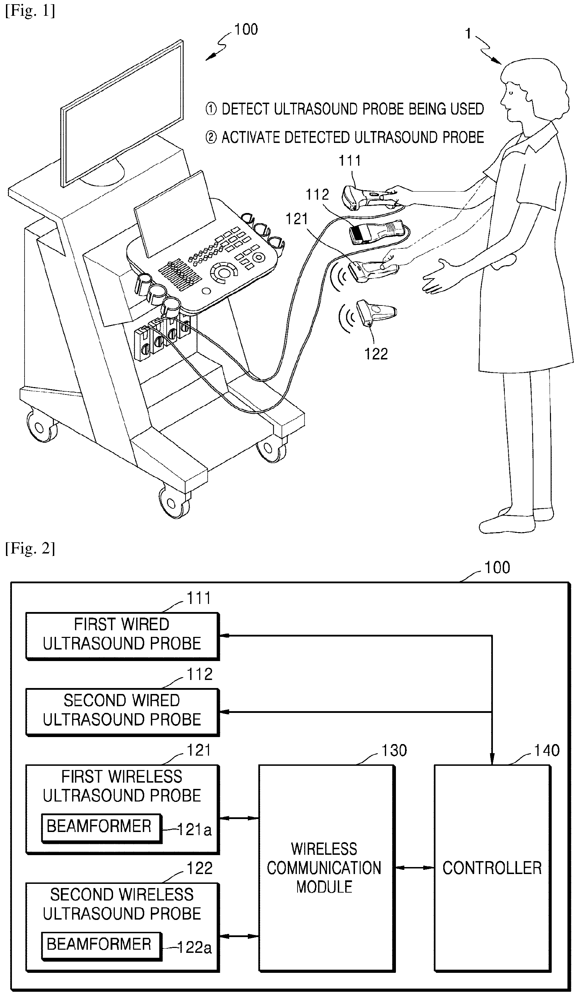

FIG. 1 is a conceptual diagram illustrating an example in which an ultrasound diagnosis apparatus 100 detects an ultrasound probe being used by a user, from among wired ultrasound probes 111 and 112 and wireless ultrasound probes 121 and 122, and activates the detected ultrasound probe, according to an embodiment.

Referring to FIG. 1, the ultrasound diagnosis apparatus 100 may be connected with at least one wired ultrasound probe and at least one wireless ultrasound probe. According to an embodiment, the ultrasound diagnosis apparatus 100 may be connected via wire with first and second wired ultrasound probes 111 and 112. Furthermore, the ultrasound diagnosis apparatus 100 may be connected with a plurality of wireless ultrasound probes including first and second wireless ultrasound probes 121 and 122 by using a wireless communication method. Although FIG. 1 shows that the number of the wired ultrasound probes 111 and 112 and the number of the wireless ultrasound probes 121 and 122 are both two (2), embodiments are not limited thereto.

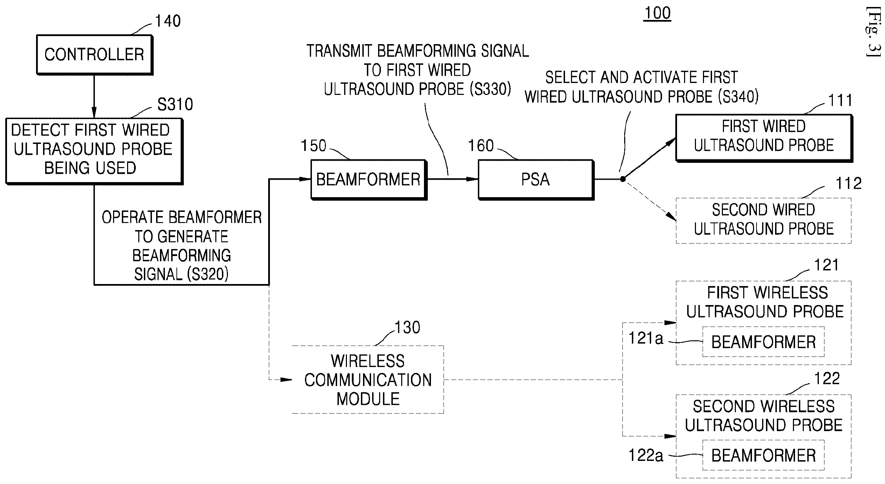

In an embodiment, the first and second wired ultrasound probes 111 and 112 may be connected to a controller (140 of FIG. 3) of the ultrasound diagnosis apparatus 100 via a probe switching assembly (PSA, 160 of FIG. 3).

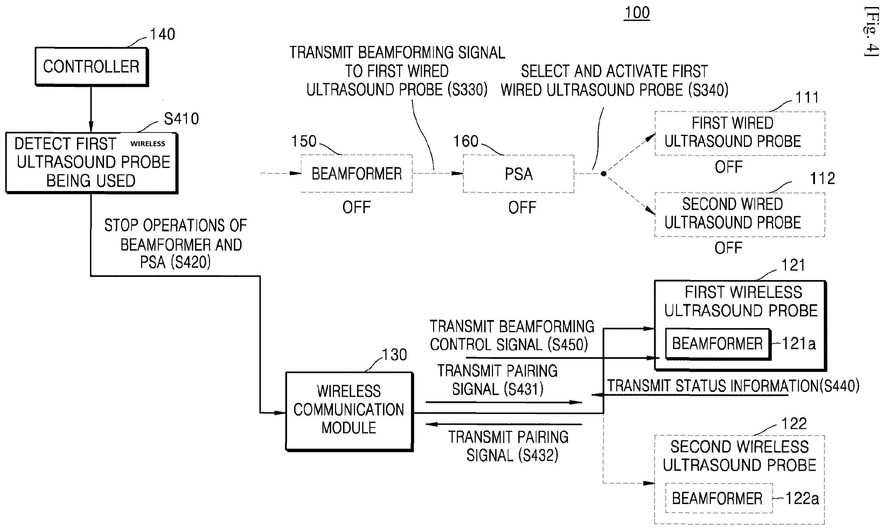

According to an embodiment, the ultrasound diagnosis apparatus 100 may be connected with the first and second wireless ultrasound probes 121 and 122 by using a wireless communication method. In this case, "connected" may mean a state in which the ultrasound diagnosis apparatus 100 is paired to use at least one of the first and second wireless ultrasound probes 121 and 122. Even when the ultrasound diagnosis apparatus 100 is connected with the first and second wireless ultrasound probes 121 and 122, it does not mean that the ultrasound diagnosis apparatus 100 may use all of the first and second wireless ultrasound probes 121 and 122 to transmit ultrasound signals to an object. "Pairing" is conceptually different from "activation", as will be described in more detail below with reference to FIG. 4.

For example, the ultrasound diagnosis apparatus 100 may be connected wirelessly with the first and second wireless ultrasound probes 121 and 122 by receiving pairing reception signals therefrom by using at least one of data communication methods including a Wireless Local Area Network (WLAN), Wireless Fidelity (Wi-Fi), Bluetooth, Zigbee, Wi-Fi Direct (WFD), Infrared Data Association (IrDA), Bluetooth Low Energy (BLE), Near Field Communication (NFC), Wireless Broadband Internet (WiBro), World Interoperability for Microwave Access (WiMAX), Shared Wireless Access Protocol (SWAP), Wireless Gigabit Alliance (WiGig), and radio frequency (RF) communication.

The first and second wireless ultrasound probes 121 and 122 may each transmit ultrasound signals to the object and receive echo signals reflected from the object to thereby produce reception signals. The first and second wireless ultrasound probes 121 and 122 may each perform image processing on the received echo signals to thereby generate ultrasound image data and then transmit the generated ultrasound image data to the controller (140 of FIGS. 2 through 4) of the ultrasound diagnosis apparatus 100.

The ultrasound diagnosis apparatus 100 may detect an ultrasound probe being used by a user 1 to examine the object among the first and second wired ultrasound probes 111 and 112 and the first and second wireless ultrasound probes 121 and 122. According to an embodiment, the ultrasound diagnosis apparatus 100 may detect a user's operation of stopping using the first wired ultrasound probe 111 and then switching probes to use the first wireless ultrasound probe 121 instead. Furthermore, the ultrasound diagnosis apparatus 100 may detect a user's operation of stopping using the first wireless ultrasound probe 121 and then switching probes to use the first wired ultrasound probe 111.

The ultrasound diagnosis apparatus 100 may detect an ultrasound probe being used by the user to examine the object and activate the detected ultrasound probe. In this case, "activation" may mean operating an ultrasound probe to transmit ultrasound signals to the object and receive ultrasound echo signals reflected from the object.

For example, when the ultrasound probe being used by the user 1 is the first wired ultrasound probe 111, the ultrasound diagnosis apparatus 100 may select the first wired ultrasound probe 111 via the PSA (160 of FIG. 3) and transmit a beamforming signal to the selected first wired ultrasound probe 111. The first wired ultrasound probe 111 may then receive the beamforming signal to transmit ultrasound signals to the object and receive ultrasound signals reflected from the object.

As another example, when the ultrasound probe being used by the user 1 is the first wireless ultrasound probe 121, the ultrasound diagnosis apparatus 100 may activate the first wireless ultrasound probe 121 and wirelessly transmit a beamforming control signal for controlling a beamformer included in the first wireless ultrasound probe 121 to the first wireless ultrasound probe 121. The first wireless ultrasound probe 121 may operate the beamformer based on the received beamforming control signal and transmit ultrasound signals generated by the beamformer to the object.

For example, when the user 1 switches the first wireless ultrasound probe 121 being used to use the first wired ultrasound probe 111 instead, the ultrasound diagnosis apparatus 100 may deactivate the first wireless ultrasound probe 121, select the first wired ultrasound probe 111 via the PSA (160 of FIG. 3), and transmit a beamforming signal to the first wired ultrasound probe 111, as will be described in more detail below with reference to FIG. 3.

As another example, when the user 1 switches the first wired ultrasound probe 111 being used to use the first wireless ultrasound probe 121, the ultrasound diagnosis apparatus 100 may deactivate the first wired ultrasound probe 111and wirelessly transmit a beamforming control signal to the beamformer included in the first wireless ultrasound probe 121, as will be described in more detail below with reference to FIG. 4.

Conventionally, ultrasound systems including only wired ultrasound probes or only one wireless ultrasound probe have been used. However, in some cases, a wired ultrasound probe and a wireless ultrasound probe need to be alternately selected for use according to characteristics of an object or a protocol necessary for diagnosing diseases of the object. A conventional ultrasound system including only a wired or wireless ultrasound probe cannot satisfy these needs. Furthermore, even in case of an ultrasound system including both wired and wireless ultrasound probes, when the user quits using the wired ultrasound probe and attempts to use the wireless ultrasound probe, the user suffers the inconvenience of having to manually pair the wireless ultrasound probe and activate the paired wireless ultrasound probe.

The ultrasound diagnosis apparatus 100 includes both at least one wired ultrasound probe (the first and second wired ultrasound probes 111 and 112) and at least one wireless ultrasound probe (the first and second wireless ultrasound probes 121 and 122) and is configured to detect an ultrasound probe being used by the user 1 to examine the object among the first and second wired ultrasound probes 111 and 112 and the first and second wireless ultrasound probes 121 and 122 and activate the detected ultrasound probe directly without a separate pairing process, thereby increasing user convenience. In particular, even when an ultrasound probe being used is switched from a wired ultrasound probe to a wireless ultrasound probe or vice versa, the ultrasound diagnosis apparatus 100 may automatically detect an ultrasound probe being used as a result of switching probes and activate the detected ultrasound probe to transmit ultrasound signals to the object and acquire ultrasound image data from the object.

FIG. 2 is a block diagram of a configuration of an ultrasound diagnosis apparatus 100 according to an embodiment.

Referring to FIG. 2, the ultrasound diagnosis apparatus 100 according to the embodiment may include first and second wired ultrasound probes 111 and 112, first and second wireless ultrasound probes 121 and 122, a wireless communication module 130, and a controller 140. Although FIG. 2 shows that the ultrasound diagnosis apparatus 100 includes the two wired ultrasound probes (the first and second wired ultrasound probes 111 and 112), this is merely an example. The ultrasound diagnosis apparatus 100 may include at least one wired ultrasound probe. Furthermore, although FIG. 2 shows that the ultrasound diagnosis apparatus 100 includes the two wireless ultrasound probes (the first and second wired ultrasound probes 121 and 122), this is merely an example. The ultrasound diagnosis apparatus 100 may include at least one wireless ultrasound probe.

According to an embodiment, the ultrasound diagnosis apparatus 100 may further include a beamformer (150 of FIG. 4) and the PSA (160 of FIG. 4).

The ultrasound diagnosis apparatus 100 may be implemented not only as a cart type apparatus but also as a portable type apparatus. Examples of portable ultrasound diagnosis apparatuses may include, but are not limited to, a picture archiving and communication system (PACS) viewer, a hand-carried cardiac ultrasound (HCU) device, a smartphone, a laptop computer, a personal digital assistant (PDA), and a tablet PC.

The first and second wired ultrasound probes 111 and 112 may each include a plurality of transducers for transmitting a beamforming signal generated by the ultrasound diagnosis apparatus 100 to an object. The plurality of transducers included in each of the first and second wired ultrasound probes 111 and 112 may receive ultrasound echo signals reflected from the object to produce reception signals. The first and second wired ultrasound probes 111 and 112 may each transmit the reflected ultrasound echo signals to the controller 140. The controller 140 may then perform analog-to-digital conversion on the received ultrasound echo signals to generate ultrasound image data and perform image processing on the ultrasound image data to obtain an ultrasound image of the object.

The first and second wireless ultrasound probes 121 and 122 may each be different types of probes having different functions, but embodiments are not limited thereto. The first and second wireless ultrasound probes 121 and 122 may each transmit ultrasound signals to the object and receive ultrasound echo signals reflected from the object to produce reception signals.

The first and second wireless ultrasound probes 121 and 122 may respectively include beamformers 121a and 122a for generating ultrasound signals that are transmitted to the object. The beamformers 121a and 122a may each receive a beamforming control signal generated by the controller 140 via the wireless communication module 130 and generate ultrasound signals that are transmitted to the object based on the received beamforming control signal.

The first and second wireless ultrasound probes 121 and 122 may each perform analog-to-digital conversion on received ultrasound echo signals and image processing of the resulting signals to thereby generate ultrasound image data regarding an object. The first and second wireless ultrasound probes 121 and 122 may each transmit generated ultrasound image data to the controller 140 via the wireless communication module 130.

The wireless communication module 130 may receive pairing reception signals from the first and second wireless ultrasound probes 121 and 122 and may be connected simultaneously with the first and second wireless ultrasound probes 121 and 122 by using a wireless communication method based on the pairing reception signals. For example, the wireless communication module 130 may simultaneously be paired wirelessly with the wireless ultrasound probes 201 through 204 by using at least one of wireless communication techniques including a WLAN, Wi-Fi, Bluetooth, Zigbee, WFD, IrDA, BLE, NFC, WiBro, WiMAX, SWAP, WiGig, and RF communication method.

According to an embodiment, the wireless communication module 130 may wirelessly receive pieces of status information regarding the first and second wireless ultrasound probes 121 and 122 based on a control signal from the controller 140. For example, status information may include at least one of a wireless communication frequency, a wireless communication connection type, an executable application, a wireless communication method, a communication status, battery charging information, a remaining battery capacity, and a remaining usability time with respect to each of the first and second wireless ultrasound probes 121 and 122.

According to an embodiment, the wireless communication module 130 may perform data communications with the first and second wireless ultrasound probes 121 and 122 by using a 60-GHz millimeter wave (mmWave) local area communication method. The wireless communication module 130 may receive raw data by using a 60-GHz mmWave wireless communication method. To acquire the raw data, the first and second wireless ultrasound probes 121 and 122 each transmit ultrasound signals to the object, process received ultrasound echo signals, and perform analog-to-digital conversion on the resulting signals.

In another embodiment, the first and second wireless ultrasound probes 121 and 122 may each perform analog-to-digital conversion on received ultrasound echo signals and perform image processing on the analog-to-digital converted signals to generate ultrasound image data. In this case, the wireless communication module 130 may receive the ultrasound image data respectively from the first and second wireless ultrasound probes 121 and 122 via Wi-Fi, WLAN, or Bluetooth.

The controller 140 may control operations of the first wired ultrasound probe 111, the second wired ultrasound probe 112, and the wireless communication module 130. In detail, the controller 140 may activate a wired ultrasound probe being selected and used by a user from among the first and second wired ultrasound probes 111 and 112. In this case, "activation" may mean operating an ultrasound probe to transmit ultrasound signals to the object and receive ultrasound echo signals reflected from the object.

Furthermore, the controller 140 may control the wireless communication module 130 to maintain wireless pairing with the first and second wireless ultrasound probes 121 and 122. Furthermore, the controller 140 may control the wireless communication module 130 to receive pieces of status information from the first and second wireless ultrasound probes 121 and 122. According to an embodiment, the controller 140 may check a status of pairing with each of the first and second wireless ultrasound probes 121 and 122 at preset time intervals. Furthermore, the controller 140 may control the wireless communication module 130 to transmit at preset time intervals a pairing signal for checking whether there is any additional wireless ultrasound probe, other than the first and second wireless ultrasound probes 121 and 122, which is to be wirelessly connected. For example, the controller 140 may control the wireless communication module 130 to transmit a pairing signal for searching for connection of an additional wireless ultrasound probe to a region near the ultrasound diagnosis apparatus 100 at 1-minute or 30-second intervals.

For example, the controller 140 may be formed as a hardware module including at least one of a central processing unit (CPU), a microprocessor, a graphic processing unit, random-access memory (RAM), and read-only memory (ROM). In an embodiment, the controller 140 may be implemented as an application processor (AP). The controller 140 may also be implemented as a hardware component such as a field-programmable gate array (FPGA) or an application-specific integrated circuit (ASIC). However, embodiments are not limited thereto, and the controller 140 may include components such as software components, object-oriented software components, class components and task components, processes, functions, attributes, procedures, subroutines, segments of program code, drivers, firmware, micro codes, circuits, data, a database, data structures, tables, arrays, and variables.

According to an embodiment, the ultrasound diagnosis apparatus 100 may further include a display configured to display a user interface (UI) indicating pieces of status information regarding the first and second wireless ultrasound probes 121 and 122 wirelessly paired thereto.

An operation of the controller 140 activating an ultrasound probe being used by a user, from among the first and second wired ultrasound probes 111 and 112 and the first and second wireless ultrasound probes 121 and 122, according to an embodiment and an operation of the controller 140 activating, when the ultrasound probe being used is switched from a wired ultrasound probe to a wireless ultrasound probe or vice versa, an ultrasound probe being used as a result of the switching according to an embodiment will now be described in detail with reference to FIGS. 3 and 4.

FIGS. 3 and 4 are flowcharts of methods of operating an ultrasound diagnosis apparatus 100.

Referring to FIGS. 3 and 4, the ultrasound diagnosis apparatus 100 includes first and second wired ultrasound probes 111 and 112, first and second wireless ultrasound probes 121 and 122, a wireless communication module 130, a controller 140, a beamformer 150, and a PSA 160. Since the first and second wired ultrasound probes 111 and 112 and the first and second wireless ultrasound probes 121 and 122, and the wireless communication module 130 correspond to their counterparts of the ultrasound diagnosis apparatus 100 described with reference to FIG. 2, descriptions that are already provided above with respect to FIG. 2 will be omitted herein.

The beamformer 150 generates a beamforming signal to be applied to a plurality of transducers, which are included in each of the first and second wired ultrasound probes 111 and 112, based on a position and a focal point of the plurality of transducers. The beamformer 150 transmits a beamforming signal to a wired ultrasound probe selected in response to a control signal from the controller 140.

The PSA 160 selects a wired ultrasound probe detected as being used by a user, from among the first and second wired ultrasound probes 111 and 112. The wired ultrasound probe connected by the PSA 160 among the first and second wired ultrasound probes 111 and 112 receives a beamforming signal from the beamformer 150 and transmits ultrasound signals to an object based on the received beamforming signal.

An operation of the controller 140 detecting the first wired ultrasound probe 111 being used by the user is now described.

The controller 140 detects the first wired ultrasound probe 111 being used (operation S310). In an embodiment, the controller 140 may detect the first wired ultrasound probe 111 being used based on a user input signal input via a user input device such as a button, mounted on the first wired ultrasound probe 111. According to an embodiment, the controller 140 may detect the first wired ultrasound probe 111 being used by using a probe information recognition method based on an RF identification (RFID), etc.

In another embodiment, the controller 140 may detect an ultrasound probe being used via a sensor built into the ultrasound probe. The sensor may detect a user input of holding one of the first and second wired ultrasound probes 111 and 112 and the first and second wireless ultrasound probes 121 and 122 in his or her hand. The sensor may then transmit ID information of the detected first wired ultrasound probe 111 to the controller 140, and the controller 140 may identify the first wired ultrasound probe 111 being used based on the received ID information.

The controller 140 generates a beamforming signal by operating the beamformer 150 (operation S320). Since the first and second wireless ultrasound probes 121 and 122 respectively includes beamformers 121a and 122a, when one of the first and second wireless ultrasound probes 121 and 122 is being used, the beamformer 150 may not operate and may be deactivated. The controller 140 may change the deactivated beamformer 150 to an activated state and control the beamformer 150 to generate a beamforming signal.

The controller 140 controls the beamformer 150 to transmit the generated beamforming signal to the first wired ultrasound probe 111 (operation S330). The controller 140 may control the beamformer 150 to transmit the beamforming signal to the first wired ultrasound probe 111 detected as being used, such that the first wired ultrasound probe 111 may emit ultrasound signals toward the object.

The controller 140 controls the PSA 160 to select and activate the first wired ultrasound probe 111 (operation S340). The PSA selects the first wired ultrasound probe 111 among the first and second wired ultrasound probes 111 and 112 based on a control signal from the controller 140 to connect a signal transmission line. The first wired ultrasound probe 111 connected by the PSA 160 may be activated to transmit ultrasound signals to the object and receive ultrasound echo signals reflected from the object.

While performing operations S310, S320, S330, and S340, the controller 140 controls the wireless communication module 130 to maintain wireless pairing respectively with the first and second wireless ultrasound probes 121 and 122. However, the controller 140 does not activate one of the first and second wireless ultrasound probes 121 and 122 to transmit ultrasound signals to the object.

Operations S310, S320, S330, and S340 are part of a method of activating the first wired ultrasound probe 111, and may be applied when an ultrasound probe being used is switched from one of the first and second wireless ultrasound probes 121 and 122 to the first wired ultrasound probe 111 in the same manner as when the first wired ultrasound probe 111 is initially selected and used.

FIG. 4 is a flowchart of a method of operating the ultrasound diagnosis apparatus 100, according to another embodiment.

An operation of the controller 140 detecting the first wireless ultrasound probe 121 being used by the user according to an embodiment will now be described in detail with reference to FIG. 4.

The controller 140 detects the first wireless ultrasound probe 121 being used (operation S410). In an embodiment, the controller 140 may detect the first wireless ultrasound probe 121 being used based on a user input signal input via a user input device such as a button, mounted on the first wireless ultrasound probe 121. According to an embodiment, the controller 140 may detect the first wireless ultrasound probe 121 being used by using a probe information recognition method based on an RFID, etc.

In another embodiment, the controller 140 may detect an ultrasound probe being used via a sensor built into the ultrasound probe. The sensor may detect a user input of holding the first wireless ultrasound probe 121 of the first and second wired ultrasound probes 121 and 122 in his or her hand. The sensor may then transmit ID information of the detected first wireless ultrasound probe 121 to the controller 140, and the controller 140 may identify the first wireless ultrasound probe 121 being used based on the received ID information.

The controller 140 stops operations of the beamformer 150 and the PSA 160 (operation S420). In an embodiment, the controller 140 may switch the beamformer 150 and the PSA 160 to an OFF state by deactivating them. When the beamformer 150 and the PSA 160 are deactivated, operations of the first and second wired ultrasound probes 111 and 112 may also be deactivated. After operation S420, no signal may be transmitted to the first and second wired ultrasound probes 111 and 112.

The controller 140 controls the wireless communication module 130 to transmit a pairing signal to the first wireless ultrasound probe 121 detected as an ultrasound probe being used (operation S431). The wireless communication module 130 may transmit a pairing signal to the first wireless ultrasound probe 121 by using a wireless communication method based on a control signal from the controller 140.

The first wireless ultrasound probe 121 may transmit a pairing signal to the wireless communication module 130 (operation S432).

In operations S431 and S432, the wireless communication module 130 may exchange a pairing signal with the first wireless ultrasound probe 121 by using at least one of wireless communication methods including a WLAN, Wi-Fi, Bluetooth, Zigbee, WFD, IrDA, BLE, NFC, WiBro, WiMAX, SWAP, WiGig, and RF communication.

The first wireless ultrasound probe 121 transmits status information to the wireless communication module 130 (operation S440). According to an embodiment, the controller 140 may control the wireless communication module 130 to receive from the first wireless ultrasound probe 121 status information including at least one of ID information, a wireless communication frequency, a connection type, an executable application, a wireless communication method, battery charging information, a remaining battery capacity, a remaining usability time, and a communication status with respect to the first wireless ultrasound probe 121. Although not shown in FIG. 4, the controller 140 may control the wireless communication module 130 to receive status information from the second wireless ultrasound probe 122 as well.

The controller 140 controls the wireless communication module 130 to transmit a beamforming control signal to the first wireless ultrasound probe 121 (operation S450). A beamforming control signal may be a signal used to control the beamformers 121a and 122b respectively included in the first and second wireless ultrasound probes 121 and 122 to perform beamforming. The controller 140 may generate beamforming control signals for controlling the beamformers 121a and 122a and control the wireless communication module 130 to transmit a beamforming control signal to the beamformer 121a included in the first wireless ultrasound probe 121 detected as being used .

Operations S410, S420, S431, S432, S440, and S450 are part of a method of activating the first wireless ultrasound probe 121, and may be applied when an ultrasound probe being used is switched from one of the first and second wired ultrasound probes 111 and 112 to the first wireless ultrasound probe 121 in the same manner as when the first wireless ultrasound probe 121 is initially selected and used.

According to the embodiments described with reference to FIGS. 3 and 4, the ultrasound diagnosis apparatus 100 is configured to detect an ultrasound probe being used by the user to examine the object among the first and second wired ultrasound probes 111 and 112 and the first and second wireless ultrasound probes 121 and 122 and automatically activate the detected ultrasound probe, thereby eliminating the need to perform unnecessary processes such as a separate wireless pairing process and termination of pairing and thus increasing user convenience.

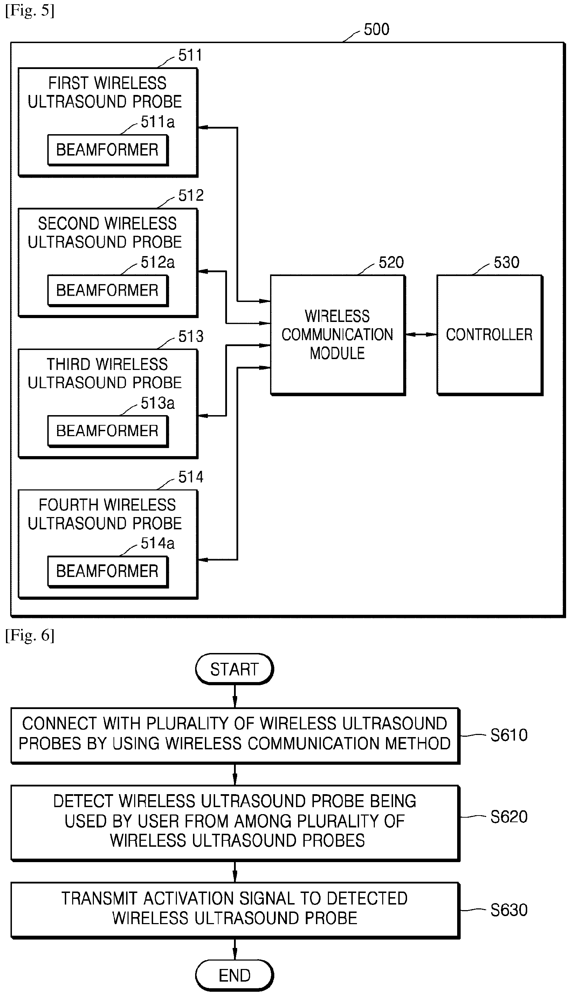

FIG. 5 is a block diagram of a configuration of an ultrasound diagnosis apparatus 500 according to another embodiment.

Referring to FIG. 5, the ultrasound diagnosis apparatus 500 may include first through fourth wireless ultrasound probes 511 through 514, a wireless communication module 520, and an a controller 530. The ultrasound diagnosis apparatus 500 of FIG. 5 does not include a wired ultrasound probe unlike the ultrasound diagnosis apparatus 200 described with reference to FIG. 2, but includes the same components as their counterparts of the ultrasound diagnosis apparatus 200. Thus, descriptions that are already provided above with respect to FIG. 2 will be omitted herein.

Although a total of four (4) wireless ultrasound probes including the first through fourth wireless ultrasound probes 511 through 514 are shown in FIG. 5, this is merely an example, and the ultrasound diagnosis apparatus 500 may include a plurality of wireless ultrasound probes.

The first through fourth wireless ultrasound probes 511 through 514 may respectively include beamformers 511a through 514a. The beamformers 511a through 514a may each generate a beamforming signal to be applied to a plurality of transducers, which are included in each of the first through fourth wireless ultrasound probes 511 through 514, based on a position and a focal point of the plurality of transducers.

The first through fourth wireless ultrasound probes 511 through 514 may each perform analog-to-digital conversion on received ultrasound echo signals and perform image processing on the analog-to-digital converted signals to thereby generate ultrasound image data. The first through fourth wireless ultrasound probes 511 through 5'4 may each transmit the generated ultrasound image data to the controller 530 via the wireless communication module 520.

The first through fourth wireless ultrasound probes 511 through 514 may each be connected with the wireless communication module 520 by using a wireless communication method. For example, the first through fourth wireless ultrasound probes 511 through 514 may be wirelessly paired with the wireless communication module 520 by using at least one of wireless communication techniques including a WLAN, Wi-Fi, Bluetooth, Zigbee, WFD, IrDA, BLE, NFC, WiBro, WiMAX, SWAP, WiGig, and RF communication method.

The wireless communication module 520 may be connected with the first through fourth wireless ultrasound probes 511 through 514 by using a wireless communication method. The wireless communication module 520 may be simultaneously paired wirelessly with the first through fourth wireless ultrasound probes 511 through 514.

According to an embodiment, the wireless communication module 520 may wirelessly receive status information regarding each of the first through fourth wireless ultrasound probes 511 through 514 based on a control signal from the controller 530. For example, the wireless communication module 520 may receive from the first wireless ultrasound probe 511 status information including at least one of ID information, a wireless communication frequency, a connection type, an executable application, a wireless communication method, battery charging information, a remaining battery capacity, a remaining usability time, and a communication status with respect to the first wireless ultrasound probe 511.

According to an embodiment, the wireless communication module 520 may perform data communication with each of the first through fourth wireless ultrasound probes 511 through 514 by using a 60-GHz mm Wave local area wireless communication method. The wireless communication module 520 may receive raw data by using a 60-GHz mmWave wireless communication method. To acquire the raw data, each of the first through fourth wireless ultrasound probes 511 through 514 transmits ultrasound signals to the object, processes received ultrasound echo signals, and performs analog-to-digital conversion on the resulting signals.

The controller 530 may detect a wireless ultrasound probe being used by the user among the first through fourth wireless ultrasound probes 511 through 514 wirelessly paired with the wireless communication module 520 and control the wireless communication module 520 to transmit an activation signal to the detected wireless ultrasound probe. According to an embodiment, the controller 530 may detect the first wireless ultrasound probe 511 being used based on a user input signal input via a user input device such as a button, mounted on the first wireless ultrasound probe 511. According to an embodiment, the controller 530 may detect the first wireless ultrasound probe 511 being used by using a probe information recognition method based on an RFID, etc.

In another embodiment, the controller 530 may detect an ultrasound probe being used via a sensor built into the ultrasound probe. The sensor may detect a user input of holding in his or her hand the first wireless ultrasound probe 511 among the first through fourth wireless ultrasound probes 511 through 514. The sensor may then transmit ID information of the detected first wireless ultrasound probe 511 to the controller 530, and the controller 530 may detect the first wireless ultrasound probe 511 being used based on the received ID information.

The controller 530 may control the wireless communication module 520 to transmit a beamforming control signal for controlling the beamformer 511a included in the detected first wireless ultrasound probe 511 to the first wireless ultrasound probe 511. The first wireless ultrasound probe 511 that has received the beamforming control signal from the controller 530 may generate ultrasound transmitting signals via the beamformer 511a and transmit the generated ultrasound transmitting signals to the object via a plurality of transducers included therein.

The controller 530 may be constructed by a hardware module including at least one of a CPU, a microprocessor, a graphic processing unit, RAM, ROM, and an AP.

Although not shown in FIG. 5, the ultrasound diagnosis apparatus 100 may further include a display configured to display a UI indicating ID information and status information regarding each of the first through fourth wireless ultrasound probes 511 through 514.

The ultrasound diagnosis apparatus 500 according to the embodiment may include a plurality of wireless ultrasound probes, i.e., the first through fourth wireless ultrasound probes 511 through 514 and may simultaneously be wirelessly paired therewith. Furthermore, the ultrasound diagnosis apparatus 500 is configured to detect a wireless ultrasound probe being used among the paired first through fourth wireless ultrasound probes 511 through 514 and automatically transmit an activation signal to the detected wireless ultrasound probe, thereby eliminating the need to perform an unnecessary pairing process and therefore increasing user convenience.

FIG. 6 is a flowchart of a method of operating an ultrasound diagnosis apparatus, according to another embodiment.

The ultrasound diagnosis apparatus is connected with a plurality of different wireless ultrasound probes by using a wireless communication method (operation S610). According to an embodiment, the plurality of wireless ultrasound probes may each be different types of wireless ultrasound probes having different functions, but are not limited thereto. The wireless ultrasound probes may be the same type of wireless ultrasound probes. In an embodiment, the ultrasound diagnosis apparatus may be connected wirelessly with the wireless ultrasound probes by using at least one of wireless communication methods including WLAN, Wi-Fi, Bluetooth, Zigbee, WFD, IrDA, BLE, NFC, WiBro, WiMAX, SWAP, WiGig, and RF communication. In operation S610, "connected" may mean a state in which the ultrasound diagnosis apparatus is paired to use at least one of the wireless ultrasound probes. In an embodiment, the ultrasound diagnosis apparatus may be paired simultaneously with the wireless ultrasound probes.

The ultrasound diagnosis apparatus detects a wireless ultrasound probe being used by a user, from among the wireless ultrasound probes (operation S620). The ultrasound diagnosis apparatus may detect a wireless ultrasound probe being used based on a user input signal input via a user input device such as a button, mounted on each of the wireless ultrasound probes. According to an embodiment, the ultrasound diagnosis apparatus may detect the wireless ultrasound probe being used by using a probe information recognition method based on an RFID, etc.

The ultrasound diagnosis apparatus transmits an activation signal to the detected wireless ultrasound probe (operation S630). In this case, "activation" is conceptually different from the "connection" or "pairing" in operation S610 and means operating the detected wireless ultrasound probe to transmit ultrasound signals to an object and receive ultrasound echo signals reflected from the object to thereby generate ultrasound image data.

According to an embodiment, each of the wireless ultrasound probes may include a beamformer. The ultrasound diagnosis apparatus may generate a beamforming control signal for controlling a beamformer included in the wireless ultrasound probe detected in operation S620 and transmit the generated beamforming control signal to the detected wireless ultrasound probe. The wireless ultrasound probe that has received the beamforming control signal may generate ultrasound transmitting signals via the beamformer and transmit the generated ultrasound transmitting signals to the object via a plurality of transducers included therein.

According to an embodiment, the activated wireless ultrasound probe may perform analog-to-digital conversion and image processing on ultrasound echo signals reflected from the object to generate ultrasound image data and transmit the generated ultrasound image data to the ultrasound diagnosis apparatus. In this case, the wireless ultrasound probe may transmit the ultrasound image data, i.e., ultrasound raw data to the ultrasound diagnosis apparatus by using a 60-GHz mmWave local area wireless communication method.

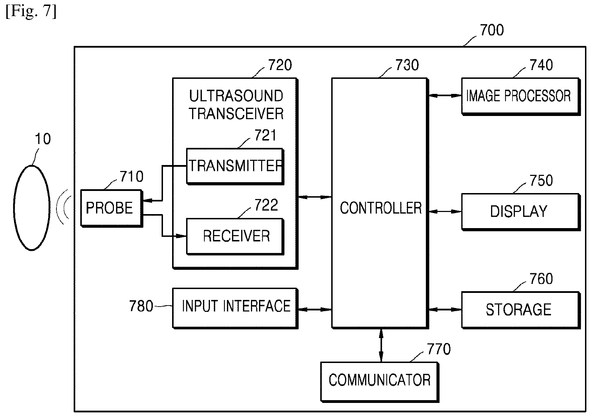

FIG. 7 is a block diagram of a configuration of an ultrasound diagnosis apparatus 700 including a wired ultrasound probe 710, according to an embodiment;

Referring to FIG. 7, the ultrasound diagnosis apparatus 700 may include the wired ultrasound probe 710, an ultrasound transceiver 720, a controller 730, an image processor 740, a display 750, a storage 760, a communicator 770, and an input interface 780.

The ultrasound diagnosis apparatus 700 may be implemented not only as a cart type apparatus but also as a portable type apparatus. Examples of a portable ultrasound diagnosis apparatus may include, but are not limited to, a smartphone, a laptop computer, a PDA, and a tablet PC.

The wired ultrasound probe 710 may include a plurality of transducers. The plurality of transducers may transmit ultrasound signals to an object 10 in response to transmitting signals received from a transmitter 721. The plurality of transducers may receive ultrasound signals reflected from the object 10 to generate reception signals. Furthermore, the wired ultrasound probe 710 may be formed integrally with the ultrasound diagnosis apparatus 700, or the wired ultrasound probe 710 and the ultrasound diagnosis apparatus 700 may be formed separately but connected to each other by wire or wirelessly. In addition, the ultrasound diagnosis apparatus 700 may include one or more wired ultrasound probes 710 according to embodiments.

The controller 730 may control the transmitter 721 to generate transmitting signals to be respectively applied to the plurality of transducers based on a position and a focal point of the plurality of transducers included in the wired ultrasound probe 710.

The controller 730 may control the ultrasound receiver 722 to generate ultrasound data by performing analog-to-digital conversion on reception signals received from the wired ultrasound probe 710 and summing the analog-to-digital converted reception signals based on a position and a focal point of the plurality of transducers.

The image processor 740 may generate an ultrasound image by using the ultrasound data generated by the ultrasound receiver 722.

The display 750 may display the generated ultrasound image and various pieces of information processed by the ultrasound diagnosis apparatus 700. The ultrasound diagnosis apparatus 700 may include one or a plurality of displays 750 according to its implemented configuration. The display 750 may be combined with a touch panel to form a touch screen.

The controller 730 may control all the operations of the ultrasound diagnosis apparatus 700 and flow of signals between the internal elements of the ultrasound diagnosis apparatus 700. The controller 730 may include a memory for storing a program or data to perform functions of the ultrasound diagnosis apparatus 700 and a processor for processing the program or data. For example, the controller 730 may control the operation of the ultrasound diagnosis apparatus 700 by receiving a control signal from the input interface 780 or an external apparatus.

The ultrasound diagnosis apparatus 700 may include the communicator 770 and may be connected to external apparatuses, for example, servers, medical apparatuses, and portable devices such as smart phones, tablet PCs, wearable devices, etc., via the communicator 770.

The communicator 770 may include at least one element that enables communication with the external apparatuses. For example, the communicator 770 may include at least one of a local area communication module, a wired communication module, and a wireless communication module.

The communicator 770 may receive a control signal and data from an external apparatus and transmit the received control signal to the controller 730 such that the controller 730 may control the ultrasound diagnosis apparatus 700 in response to the received control signal.

The controller 730 may also transmit a control signal to the external apparatus via the communicator 770 such that the external apparatus may be controlled in response to the control signal from the controller 730.

For example, the external apparatus may process data of the external apparatus in response to the control signal from the controller 730 received via the communicator 770.

A program for controlling the ultrasound diagnosis apparatus 700 may be installed in the external apparatus. The program may include command languages for performing part of operation of the controller 730 or the entire operation thereof.

The program may be pre-installed in the external apparatus or may be installed by a user of the external apparatus by downloading the program from a server that provides applications. The server that provides applications may include a recording medium where the program is stored.

The storage 760 may store various pieces of data or programs for driving and controlling the ultrasound diagnosis apparatus 700, input and/or output ultrasound data, obtained ultrasound images, etc.

The input interface 780 may receive a user input for controlling the ultrasound diagnosis apparatus 700. For example, the user input may include an input for manipulating a button, a keypad, a mouse, a trackball, a jog switch, or a knob, an input for touching a touchpad or a touch screen, a voice input, a motion input, and an input of biometric information such as iris recognition or fingerprint recognition, but embodiments are not limited thereto.

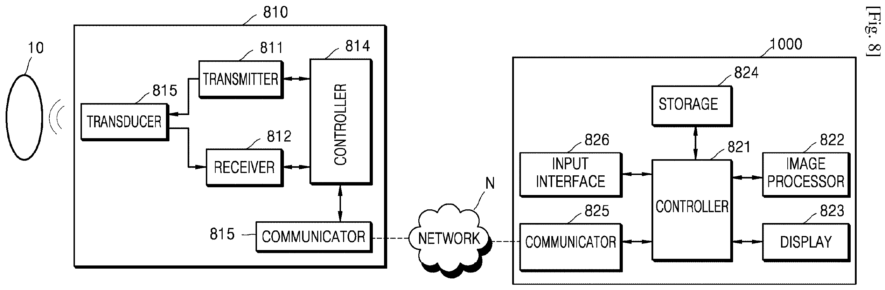

FIG. 8 is a block diagram of a configuration of an ultrasound system 800 including a wireless ultrasound probe 810, according to an embodiment.

Referring to FIG 8, an ultrasound diagnosis apparatus 820 may be connected with the wireless ultrasound probe 800 via a network N.

The wireless ultrasound probe 810 may include a transmitter 811, a receiver 812, a transducer 813, a controller 814, and a communicator 815. Although FIG. 8 shows that the wireless ultrasound probe 810 includes both the transmitter 811 and the receiver 812, according to an implemented configuration, the wireless ultrasound probe 810 may include some of the components of the transmitter 811 and the receiver 812 while the ultrasound diagnosis apparatus 820 may also include some of the components thereof.

The transducer 813 may include a plurality of transducer elements. The plurality of transducer elements may transmit ultrasound signals to an object 10 in response to transmitting signals received from the transmitter 811. The transducer elements may receive ultrasound signals reflected from the object 10 to generate reception signals.

The controller 814 controls the transmitter 811 to generate transmitting signals to be respectively applied to the transducer elements based on positions and focal points of the transducer elements.

The controller 814 controls the receiver 812 to generate ultrasound data by performing analog-to-digital conversion on the reception signals received from the transducer 813 and summing the analog-to-digital converted reception signals based on a position and a focal point of the transducer elements.

The communicator 815 may wirelessly transmit the generated ultrasound data or ultrasound image to the ultrasound diagnosis apparatus 820 via a wireless network. Alternatively, the communicator 815 may receive a control signal and data from the ultrasound diagnosis apparatus 820.

The ultrasound diagnosis apparatus 820 may receive ultrasound data or an ultrasound image from the wireless ultrasound probe 810. The ultrasound diagnosis apparatus 820 may include a controller 821, an image processor 822, a display 823, and a storage 824, a communicator 825, and an input interface 826.

The controller 821 may control all operations of the ultrasound diagnosis apparatus 820 and flow of signals between the internal elements of the ultrasound diagnosis apparatus 820. The controller 821 may include a memory for storing a program or data to perform functions of the ultrasound diagnosis apparatus 820 and a processor for processing the program or data. Furthermore, the controller 821 may control the operation of the ultrasound diagnosis apparatus 820 by receiving a control signal from the input interface 826 or an external apparatus.

The ultrasound diagnosis apparatus 820 may include the communicator 825 and may be connected to external apparatuses, for example, servers, medical apparatuses, and portable devices such as smart phones, tablet PCs, wearable devices, etc., via the communicator 825.

The communicator 825 may include at least one element capable of communicating with the external apparatuses. For example, the communicator 825 may include at least one of a local area communication module, a wired communication module, and a wireless communication module.

The communicator 825 may receive a control signal and data from an external apparatus and transmit the received control signal to the controller 821 such that the controller 821 may control the ultrasound diagnosis apparatus 820 in response to the received control signal.

Alternatively, the controller 821 may transmit a control signal to the external apparatus via the communicator 825 to control the external apparatus in response to the control signal from the controller 821.

For example, the external apparatus may process data from the external apparatus in response to the control signal from the controller 821 received via the communicator 825.

A program for controlling the ultrasound diagnosis apparatus 820 may be installed in the external apparatus. The program may include command languages for performing part of operation of the controller 821 or the entire operation thereof.

The program may be pre-installed in the external apparatus or may be installed by a user of the external apparatus by downloading the program from a server that provides applications. The server that provides applications may include a recording medium on which the program is stored.

The image processor 822 may generate an ultrasound image by using ultrasound data received from the wireless ultrasound probe 810.

The display 823 may display an ultrasound image received from the wireless ultrasound probe 810 and an ultrasound image generated by the ultrasound diagnosis apparatus 820. The ultrasound diagnosis apparatus 820 may include two or more displays 823 according to its implemented configuration. Furthermore, the display 823 may be combined with a touch panel to form a touch screen.

The storage 824 may store various pieces of data or programs for driving and controlling the ultrasound diagnosis apparatus 820, input and/or output ultrasound data, ultrasound images, etc.

The input interface 826 receives a user input for controlling the ultrasound diagnosis apparatus 820. For example, the user input may include an input for manipulating a button, a keypad, a mouse, a trackball, a jog switch, or a knob, an input for touching a touchpad or a touch screen, a voice input, a motion input, and an input of biometric information such as iris recognition or fingerprint recognition, but embodiments are not limited thereto.

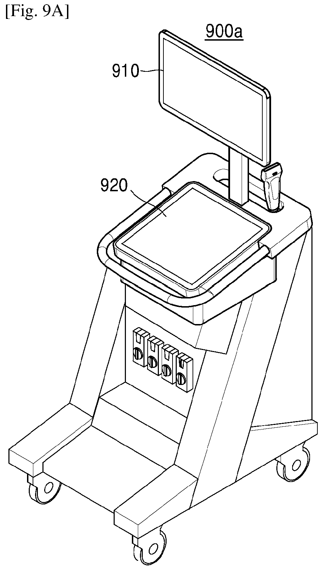

FIGS. 9A, 9B, and 9C are diagrams illustrating ultrasound diagnosis apparatus according to an exemplary embodiment.

Referring to FIGS. 9A and 9B, the ultrasound diagnosis apparatuses 900a and 900b may include a main display 910 and a sub-display 920. At least one among the main display 910 and the sub-display 920 may include a touch screen. The main display 910 and the sub-display 920 may display ultrasound images and/or various information processed by the ultrasound diagnosis apparatuses 900a and 900b. The main display 910 and the sub-display 920 may provide graphical user interfaces (GUI), thereby receiving user's inputs of data to control the ultrasound diagnosis apparatuses 900a and 900b. For example, the main display 910 may display an ultrasound image and the sub-display 920 may display a control panel to control display of the ultrasound image as a GUI. The sub-display 920 may receive an input of data to control the display of an image through the control panel displayed as a GUI. The ultrasound diagnosis apparatuses 900a and 900b may control the display of the ultrasound image on the main display 910 by using the input control data.

Referring to FIG. 9B, the ultrasound diagnosis apparatus 900b may include a control panel 930. The control panel 930 may include buttons, trackballs, jog switches, or knobs, and may receive data to control the ultrasound diagnosis apparatus 900b from the user. For example, the control panel 930 may include a time gain compensation (TGC) button 941 and a freeze button 942. The TGC button 941 is to set a TGC value for each depth of an ultrasound image. Also, when an input of the freeze button 942 is detected during scanning an ultrasound image, the ultrasound diagnosis apparatus 900b may keep displaying a frame image at that time point.

## Claims

1. An ultrasound diagnosis apparatus (100), comprising:
at least one wired ultrasound probe (111, 112) connected via wire to the ultrasound diagnosis apparatus;
a plurality of wireless ultrasound probes (121, 122) connected to the ultrasound diagnosis apparatus (100) by using wireless communication;
a wireless communication module (130) configured to receive a pairing reception signal from each of the plurality of wireless ultrasound probes (121, 122) to thereby be connected with the plurality of wireless ultrasound probes (121, 122) by using a wireless communication method and to transmit and receive a beamforming control signal and ultrasound image data to and from each of the plurality of wireless ultrasound probes (121, 122);
a beamformer (150) configured to generate a beamforming signal to be applied to each of a plurality of transducers included in each of at least one wired ultrasound probe (111, 112), based on a position and a focal point of the plurality of transducers;
a probe switching assembly (160) configured to select a wired ultrasound probe from among the at least one wired ultrasound probe (111, 112) and activate the selected wired ultrasound probe; and
a controller (140) configured to detect an ultrasound probe being used by a user to examine an object, from among the at least one wired ultrasound probe and the plurality of wireless ultrasound probes (121, 122), and activate the detected ultrasound probe
**CHARACTERIZED IN THAT**
the wireless communication module (130) is simultaneously paired with the plurality of wireless ultrasound probes (121, 122), and
the controller (140) is further configured to:
when the ultrasound probe detected as being used is a first wired ultrasound probe (111) among the at least one wired ultrasound probe (111, 112), control the probe switching assembly (160) to select and activate the first wired ultrasound probe (111), control the beamformer (150) to generate the beamforming signal and transmit the generated beamforming signal to the first wired ultrasound probe (111), and control the wireless communication module (130) to maintain wireless pairing with the plurality of wireless ultrasound probes (121, 122).

2. The ultrasound diagnosis apparatus of claim 1, wherein the controller (140) is further configured to stop operations of the beamformer (150) and the probe switching assembly (160), when the ultrasound probe detected as being used is switched from the first wired ultrasound probe activated among the at least one wired ultrasound probe (111, 112) to one of the plurality of wireless ultrasound probes (121, 122).

3. The ultrasound diagnosis apparatus of claim 2, wherein the controller (140) is further configured to resume, when the ultrasound probe detected as being used is switched from the wireless ultrasound probe (121, 122) to the first wired ultrasound probe (111), the operations of the beamformer (150) and the probe switching assembly (160), control the probe switching assembly (160) to activate the first wired ultrasound probe (111), and control the beamformer to transmit the beamforming signal to the first wired ultrasound probe (111).

4. The ultrasound diagnosis apparatus of claim 1, wherein the controller (140) is further configured to control the wireless communication module (130) to transmit, when the ultrasound probe detected as being used is a first wireless ultrasound probe (121, 122), a beamforming control signal for controlling a beamformer included in the first wireless ultrasound probe (121, 122) to the first wireless ultrasound probe.

5. The ultrasound diagnosis apparatus of claim 1, wherein the controller (140) is further configured to control the wireless communication module (130) to receive, from the plurality of wireless ultrasound probes (121, 122), status information including at least one of identification information, a wireless communication frequency, a connection type, an executable application, a wireless communication method, battery charging information, a remaining battery capacity, a remaining usability time, and a communication status with respect to each of the plurality of wireless ultrasound probes (121, 122).

6. The ultrasound diagnosis apparatus of claim 1, wherein the wireless communication module (130) is connected with the plurality of wireless ultrasound probes (121, 122) by using at least one of wireless communication methods comprising a Wireless Local Area Network (WLAN), wireless fidelity (Wi-Fi), Bluetooth, Zigbee, Wi-Fi Direct (WFD), Infrared Data Association (IrDA), Bluetooth Low Energy (BLE), Near Field Communication (NFC), Wireless Broadband Internet (WiBro), World Interoperability for Microwave Access (WiMAX), Shared Wireless Access Protocol (SWAP), Wireless Gigabit Alliance (WiGig), and radio frequency (RF) communication.

7. The ultrasound diagnosis apparatus of claim 1, wherein the controller (140) is further configured to check a status of wireless connection between the ultrasound diagnosis apparatus and the plurality of wireless ultrasound probes (121, 122) at preset time intervals.

8. A method of operating an ultrasound diagnosis apparatus comprising at least one wired ultrasound probe, plurality of wireless ultrasound probes, a wireless communication module (130) configured to receive a pairing reception signal from each of the plurality of wireless ultrasound probes (121, 122) to thereby be connected with the plurality of wireless ultrasound probes (121, 122) by using a wireless communication method, and to transmit and receive beamforming control signal and ultrasound image data to and from each of the plurality of wireless ultrasound probes; a beamformer (150) configured to generate a beamforming signal to be applied to each of a plurality of transducers included in each of at least one wired ultrasound probe (111, 112) based on a position and a focal point of the plurality of transducers, and a probe switching assembly (160) configured to select a wired ultrasound probe from among the at least one wired ultrasound probe (111, 112) and activate the selected wired ultrasound probe, and a controller, the method comprising:
connecting the ultrasound diagnosis apparatus with the plurality of wireless ultrasound probes by using a wireless communication method;
simultaneously pairing the wireless communication module with the plurality of wireless ultrasound probes;
detecting an ultrasound probe being used by a user to examine an object, from among the at least one wired ultrasound probe and the plurality of wireless ultrasound probes; and
when the ultrasound probe detected as being used is a first wired ultrasound probe (111) among the at least one wired ultrasound probe (111, 112), controlling the probe switching assembly (160) to select and activate the first wired ultrasound probe (111), controlling the beamformer (150) to generate a beamforming signal and transmit the generated beamforming signal to the first wired ultrasound probe (111), and controlling the wireless communication module (130) to maintain wireless pairing with the plurality of wireless ultrasound probes (121, 122).

9. The method of claim 8, further comprising stopping an operation of the probe switching assembly (160) and the beamformer (150) when the ultrasound probe detected as being used is switched from the first wired ultrasound probe to one of the plurality of wireless ultrasound probes (121, 122).

10. The method of claim 9, further comprising:
resuming, when the ultrasound probe detected as being used is switched from the wireless ultrasound probe to a second wired ultrasound probe, the operation of the probe switching assembly (160) to activate the second wired ultrasound probe; and
controlling the beamformer (150) to transmit the beamforming signal to the second wired ultrasound probe.

11. A computer-readable recording medium having recorded thereon a program for executing the method of claim 8 on a computer.

## Patentansprüche

1. Ultraschalldiagnosevorrichtung (100), die Folgendes umfasst:
zumindest eine drahtgebundene Ultraschallsonde (111, 112), die über ein Kabel mit der Ultraschalldiagnosevorrichtung verbunden ist;
eine Vielzahl von drahtlosen Ultraschallsonden (121, 122), die über eine drahtlose Kommunikation mit der Ultraschalldiagnosevorrichtung (100) verbunden sind;
ein drahtloses Kommunikationsmodul (130), das konfiguriert ist, um ein Paarungsempfangssignal von jeder der Vielzahl von drahtlosen Ultraschallsonden (121, 122) zu empfangen, um dadurch unter Verwendung eines drahtlosen Kommunikationsverfahrens mit der Vielzahl von drahtlosen Ultraschallsonden (121, 122) verbunden zu werden und um ein Strahlformungssteuersignal sowie Ultraschallbilddaten zu jeder der Vielzahl von drahtlosen Ultraschallsonden zu übertragen und von diesen zu empfangen (121, 122);
einen Strahlformer (150), der konfiguriert ist, um ein Strahlformungssignal zu erzeugen, das an jeden einer Vielzahl von Wandlern angelegt wird, die in jeder von zumindest einer drahtgebundenen Ultraschallsonde (111, 112) enthalten sind, basierend auf einer Position und einem Fokalpunkt der Vielzahl von Wandlern;
eine Sondenschaltanordnung (160), die konfiguriert ist, um eine drahtgebundene Ultraschallsonde unter der zumindest einen drahtgebundenen Ultraschallsonde (111, 112) auszuwählen und die ausgewählte drahtgebundene Ultraschallsonde zu aktivieren; und
eine Steuerung (140), die konfiguriert ist, um eine Ultraschallsonde, die von einem Benutzer verwendet wird, um ein Objekt zu untersuchen, unter der zumindest einen drahtgebundenen Ultraschallsonde und der Vielzahl von drahtlosen Ultraschallsonden (121, 122) zu erfassen und die erfasste Ultraschallsonde zu aktivieren,
**DADURCH GEKENNZEICHNET, DASS**
das drahtlose Kommunikationsmodul (130) gleichzeitig mit der Vielzahl von drahtlosen Ultraschallsonden (121, 122) gepaart ist, und
die Steuerung (140) ferner zu Folgendem konfiguriert ist:
wenn es sich bei der als verwendet erfassten Ultraschallsonde um eine erste drahtgebundene Ultraschallsonde (111) unter der zumindest einen drahtgebundenen Ultraschallsonde (111, 112) handelt, Steuern der Sondenschaltanordnung (160) zum Auswählen und Aktivieren der ersten drahtgebundenen Ultraschallsonde (111), Steuern des Strahlformers (150) zum Erzeugen des Strahlformungssignals und Übertragen des erzeugten Strahlformungssignals an die erste drahtgebundene Ultraschallsonde (111) sowie Steuern des drahtlosen Kommunikationsmoduls (130) zum Aufrechterhalten der drahtlosen Paarung mit der Vielzahl von drahtlosen Ultraschallsonden (121, 122).

2. Ultraschalldiagnosevorrichtung nach Anspruch 1, wobei die Steuerung (140) ferner konfiguriert ist, um den Betrieb des Strahlformers (150) und der Sondenschaltanordnung (160) zu stoppen, wenn die als verwendet erfasste Ultraschallsonde von der ersten drahtgebundenen Ultraschallsonde, die unter der zumindest einen drahtgebundenen Ultraschallsonde (111, 112) aktiviert wurde, auf eine der Vielzahl von drahtlosen Ultraschallsonden (121, 122) umgeschaltet wird.

3. Ultraschalldiagnosevorrichtung nach Anspruch 2, wobei die Steuerung (140) ferner konfiguriert ist, um, wenn die als verwendet erfasste Ultraschallsonde von der drahtlosen Ultraschallsonden (121, 122) auf die erste drahtgebundene Ultraschallsonde (111) umgeschaltet wird, den Betrieb des Strahlformers (150) und der Sondenschaltanordnung (160) wieder aufzunehmen, die Sondenschaltanordnung (160) so zu steuern, dass sie die erste drahtgebundene Ultraschallsonde (111) aktiviert, und den Strahlformer so zu steuern, dass er das Strahlformungssignal an die erste drahtgebundene Ultraschallsonde (111) überträgt.

4. Ultraschalldiagnosevorrichtung nach Anspruch 1, wobei die Steuerung (140) ferner konfiguriert ist, um das drahtlose Kommunikationsmodul (130) so zu steuern, dass es, wenn es sich bei der als verwendet erfassten Ultraschallsonde um eine erste drahtlose Ultraschallsonde (121, 122) handelt, ein Strahlformungssteuersignal zum Steuern eines in der ersten drahtlosen Ultraschallsonde (121, 122) enthaltenen Strahlformers an die erste drahtlose Ultraschallsonde überträgt.

5. Ultraschalldiagnosevorrichtung nach Anspruch 1, wobei die Steuerung (140) ferner konfiguriert ist, um das drahtlose Kommunikationsmodul (130) so zu steuern, dass es von der Vielzahl von drahtlosen Ultraschallsonden (121, 122) Statusinformationen empfängt, die zumindest eine der folgenden Informationen enthalten: Identifizierungsinformationen, eine drahtlose Kommunikationsfrequenz, einen Verbindungstyp, eine ausführbare Anwendung, ein drahtloses Kommunikationsverfahren, Batterieladeinformationen, eine verbleibende Batteriekapazität, eine verbleibende Verwendbarkeitszeit und einen Kommunikationsstatus in Bezug auf jede der Vielzahl von drahtlosen Ultraschallsonden (121, 122).

6. Ultraschalldiagnosevorrichtung nach Anspruch 1, wobei das drahtlose Kommunikationsmodul (130) mit der Vielzahl von drahtlosen Ultraschallsonden (121, 122) verbunden ist, und zwar unter Verwendung von zumindest einem drahtlosen Kommunikationsverfahren, das die Folgenden umfasst: Wireless Local Area Network (WLAN), Wireless Fidelity (Wi-Fi), Bluetooth, Zigbee, Wi-Fi Direct (WFD), Infrared Data Association (IrDA), Bluetooth Low Energy (BLE), Near Field Communication (NFC), Wireless Broadband Internet (WiBro), World Interoperability for Microwave Access (WiMAX), Shared Wireless Access Protocol (SWAP), Wireless Gigabit Alliance (WiGig) und Radiofrequenz (RF)-Kommunikation.

7. Ultraschalldiagnosevorrichtung nach Anspruch 1, wobei die Steuerung (140) ferner konfiguriert ist, um einen Status einer drahtlosen Verbindung zwischen der Ultraschalldiagnosevorrichtung und der Vielzahl von drahtlosen Ultraschallsonden (121, 122) in vordefinierten Zeitintervallen zu überprüfen.

8. Verfahren zum Betreiben einer Ultraschalldiagnosevorrichtung, die Folgendes umfasst: zumindest eine drahtgebundene Ultraschallsonde, eine Vielzahl von drahtlosen Ultraschallsonden, ein drahtloses Kommunikationsmodul (130), das konfiguriert ist, um ein Paarungsempfangssignal von jeder der Vielzahl von drahtlosen Ultraschallsonden (121, 122) zu empfangen, um dadurch unter Verwendung eines drahtlosen Kommunikationsverfahrens mit der Vielzahl von drahtlosen Ultraschallsonden (121, 122) verbunden zu werden und um ein Strahlformungssteuersignal sowie Ultraschallbilddaten zu jeder der Vielzahl von drahtlosen Ultraschallsonden zu übertragen und von diesen zu empfangen; einen Strahlformer (150), der konfiguriert ist, um ein Strahlformungssignal zu erzeugen, das an jeden einer Vielzahl von Wandlern angelegt wird, die in jeder der zumindest einen drahtgebundenen Ultraschallsonden (111, 112) enthalten sind, basierend auf einer Position und einem Fokalpunkt der Vielzahl von Wandlern, und eine Sondenschaltanordnung (160), die konfiguriert ist, um eine drahtgebundene Ultraschallsonde unter der zumindest einen drahtgebundenen Ultraschallsonde (111, 112) auszuwählen und die ausgewählte drahtgebundene Ultraschallsonde zu aktivieren, und eine Steuerung, wobei das Verfahren Folgendes umfasst:
Verbinden der Ultraschalldiagnosevorrichtung mit der Vielzahl von drahtlosen Ultraschallsonden unter Verwendung eines drahtlosen Kommunikationsverfahrens;
gleichzeitiges Paaren des drahtlosen Kommunikationsmoduls mit der Vielzahl von drahtlosen Ultraschallsonden;
Erfassen einer Ultraschallsonde, die von einem Benutzer verwendet wird, um ein Objekt zu untersuchen, unter der zumindest einen drahtgebundenen Ultraschallsonde und der Vielzahl von drahtlosen Ultraschallsonden; und
wenn es sich bei der als verwendet erfassten Ultraschallsonde um eine erste drahtgebundene Ultraschallsonde (111) unter der zumindest einen drahtgebundenen Ultraschallsonde (111, 112) handelt, Steuern der Sondenschaltanordnung (160) zum Auswählen und Aktivieren der ersten drahtgebundenen Ultraschallsonde (111), Steuern des Strahlformers (150) zum Erzeugen eines Strahlformungssignals und Übertragen des erzeugten Strahlformungssignals an die erste drahtgebundene Ultraschallsonde (111) sowie Steuern des drahtlosen Kommunikationsmoduls (130) zum Aufrechterhalten der drahtlosen Paarung mit der Vielzahl von drahtlosen Ultraschallsonden (121, 122).

9. Verfahren nach Anspruch 8, ferner umfassend das Stoppen eines Betriebs der Sondenschaltanordnung (160) und des Strahlformers (150), wenn die als verwendet erfasste Ultraschallsonde von der ersten drahtgebundenen Ultraschallsonde auf eine der Vielzahl von drahtlosen Ultraschallsonden (121, 122) umgeschaltet wird.

10. Verfahren nach Anspruch 9, ferner umfassend:
wenn die als verwendet erfasste Ultraschallsonde von der drahtlosen Ultraschallsonde auf eine zweite drahtgebundene Ultraschallsonde umgeschaltet wird, Wiederaufnehmen des Betriebs der Sondenschaltanordnung (160), um die zweite drahtgebundene Ultraschallsonde zu aktivieren; und
Steuern des Strahlformers (150), um das Strahlformungssignal an die zweite drahtgebundene Ultraschallsonde zu übertragen.

11. Computerlesbares Aufzeichnungsmedium, auf dem ein Programm zum Ausführen des Verfahrens nach Anspruch 8 auf einem Computer aufgezeichnet ist.

## Revendications

1. Appareil d'échographie diagnostique (100), comprenant :
au moins une sonde à ultrasons filaire (111, 112) reliée par un fil à l'appareil d'échographie diagnostique,
une pluralité de sondes à ultrasons sans fil (121, 122) reliées à l'appareil d'échographie diagnostique (100) par communication sans fil,
un module de communication sans fil (130) conçu pour recevoir un signal de réception d'appariement en provenance de chacune des sondes à ultrasons sans fil (121, 122) pour être ainsi relié à la pluralité de sondes à ultrasons sans fil (121, 122) au moyen d'un mode de communication sans fil et pour émettre et recevoir un signal de commande de formation de faisceau et des données d'image échographique vis-à-vis de de chacune des sondes à ultrasons sans fil (121, 122),
un formateur de faisceau (150) conçu pour générer un signal de formation de faisceau à appliquer à chaque transducteur d'une pluralité de transducteurs présents dans chaque sonde d'au moins une sonde à ultrasons filaire (111, 112), compte tenu de la position et du point focal de la pluralité de transducteurs,
un ensemble de branchement de sonde (160) conçu pour sélectionner une sonde à ultrasons filaire parmi l'au moins une sonde à ultrasons filaire (111, 112) et activer la sonde à ultrasons filaire sélectionnée, et
un organe de commande (140) conçu pour détecter l'utilisation d'une sonde à ultrasons par un utilisateur pour examiner un objet, parmi l'au moins une sonde à ultrasons filaire et la pluralité de sondes à ultrasons sans fil (121, 122), et activer la sonde à ultrasons détectée ;
**CARACTÉRISÉ EN CE QUE**
le module de communication sans fil (130) est appairé simultanément à la pluralité de sondes à ultrasons sans fil (121, 122), et
l'organe de commande (140) est en outre conçu pour :
lorsque la sonde à ultrasons dont l'utilisation est détectée, parmi l'au moins une sonde à ultrasons filaire (111, 112), est une première sonde à ultrasons filaire (111), commander l'ensemble de branchement de sonde (160) pour sélectionner et activer la première sonde à ultrasons filaire (111), commander le formateur de faisceau (150) pour générer le signal de formation de faisceau et émettre le signal de formation de faisceau généré à destination de la première sonde à ultrasons filaire (111), et commander le module de communication sans fil (130) pour maintenir l'appariement sans fil avec la pluralité de sondes à ultrasons sans fil (121, 122).

2. Appareil d'échographie diagnostique selon la revendication 1, dans lequel l'organe de commande (140) est en outre conçu pour interrompre l'exploitation du formateur de faisceaux (150) et de l'ensemble de branchement de sonde (160) lorsque la sonde à ultrasons dont l'utilisation est détectée passe de la première sonde à ultrasons filaire activée parmi l'au moins une sonde à ultrasons filaire (111, 112) à l'une des sondes à ultrasons sans fil (121, 122).

3. Appareil d'échographie diagnostique selon la revendication 2, dans lequel l'organe de commande (140) est en outre conçu, lorsque la sonde à ultrasons dont l'utilisation est détectée passe de la sonde à ultrasons sans fil (121, 122) à la première sonde à ultrasons filaire (111), pour reprendre l'exploitation du formateur de faisceaux (150) et de l'ensemble de branchement de sonde (160), commander l'ensemble de branchement de sonde (160) pour activer la première sonde à ultrasons filaire (111), et commander le formateur de faisceaux de façon qu'il émette le signal de formation de faisceaux à destination de la première sonde à ultrasons filaire (111).

4. Appareil d'échographie diagnostique selon la revendication 1, dans lequel l'organe de commande (140) est en outre conçu pour commander le module de communication sans fil (130) de façon qu'il émette, lorsque la sonde à ultrasons dont l'utilisation est détectée est une première sonde à ultrasons sans fil (121, 122), un signal de commande de formation de faisceau visant à commander un formateur de faisceau présent dans la première sonde à ultrasons sans fil (121, 122), à destination de la première sonde à ultrasons sans fil.

5. Appareil d'échographie diagnostique selon la revendication 1, dans lequel l'organe de commande (140) est en outre conçu pour commander le module de communication sans fil (130) de façon qu'il reçoive, en provenance de la pluralité de sondes à ultrasons sans fil (121, 122), des informations d'état comprenant au moins l'une des informations suivantes : informations d'identification, fréquence de communication sans fil, type de liaison, application exécutable, mode de communication sans fil, informations de charge de batterie, capacité de batterie restante, durée d'utilisation restante et état de communication par rapport à chacune des sondes à ultrasons sans fil (121, 122).

6. Appareil d'échographie diagnostique selon la revendication 1, dans lequel le module de communication sans fil (130) est relié à la pluralité de sondes à ultrasons sans fil (121, 122) au moyen d'au moins un mode de communication sans fil parmi lesquels : communication par réseau local sans fil (WLAN, wireless local area network), Wi-Fi, Bluetooth, Zigbee, Wi-Fi Direct, IrDA (Infrared Data Association), Bluetooth basse consommation (BLE, Bluetooth Low Energy), communication en champ proche (CCP), WiBro (Wireless Broadband Internet), WiMAX (World Interoperability for Microwave Access), SWAP (Shared Wireless Access Protocol), WiGig (Wireless Gigabit Alliance) et communication par radiofréquence (RF).

7. Appareil d'échographie diagnostique selon la revendication 1, dans lequel l'organe de commande (140) est en outre conçu pour vérifier l'état de liaison sans fil entre l'appareil d'échographie diagnostique et la pluralité de sondes à ultrasons sans fil (121, 122) à intervalles de temps prédéfinis.

8. Procédé d'exploitation d'un appareil d'échographie diagnostique comprenant au moins une sonde à ultrasons filaire ; une pluralité de sondes à ultrasons sans fil ; un module de communication sans fil (130) conçu pour recevoir un signal de réception d'appariement en provenance de chacune des sondes à ultrasons sans fil (121, 122) pour être ainsi relié à la pluralité de sondes à ultrasons sans fil (121, 122) au moyen d'un mode de communication sans fil, et pour émettre et recevoir un signal de commande de formation de faisceau et des données d'image échographique vis-à-vis de chacune des sondes à ultrasons sans fil ; un formateur de faisceau (150) conçu pour générer un signal de formation de faisceau à appliquer à chaque transducteur d'une pluralité de transducteurs présents dans chaque sonde d'au moins une sonde à ultrasons filaire (111, 112) compte tenu de la position et du point focal de la pluralité de transducteurs ; un ensemble de branchement de sonde (160) conçu pour sélectionner une sonde à ultrasons filaire parmi l'au moins une sonde à ultrasons filaire (111, 112) et activer la sonde à ultrasons filaire sélectionnée ; et un organe de commande ; le procédé comprenant :
la liaison de l'appareil d'échographie diagnostique à la pluralité de sondes à ultrasons sans fil au moyen d'un mode de communication sans fil,
l'appariement simultané du module de communication sans fil à la pluralité de sondes à ultrasons sans fil,
la détection de l'utilisation d'une sonde à ultrasons par un utilisateur pour examiner un objet, parmi l'au moins une sonde à ultrasons filaire et la pluralité de sondes à ultrasons sans fil, et
lorsque la sonde à ultrasons dont l'utilisation est détectée, parmi l'au moins une sonde à ultrasons filaire (111, 112), est une première sonde à ultrasons filaire (111), la commande de l'ensemble de branchement de sonde (160) pour sélectionner et activer la première sonde à ultrasons filaire (111), la commande du formateur de faisceau (150) pour générer un signal de formation de faisceau et émettre le signal de formation de faisceau généré à destination de la première sonde à ultrasons filaire (111), et la commande du module de communication sans fil (130) pour maintenir l'appariement sans fil avec la pluralité de sondes à ultrasons sans fil (121, 122).

9. Procédé selon la revendication 8, comprenant en outre l'interruption de l'exploitation de l'ensemble de branchement de sonde (160) et du formateur de faisceau (150) lorsque la sonde à ultrasons dont l'utilisation est détectée passe de la première sonde à ultrasons filaire à l'une des sondes à ultrasons sans fil (121, 122).

10. Procédé selon la revendication 9, comprenant en outre :
lorsque la sonde à ultrasons dont l'utilisation est détectée passe de la sonde à ultrasons sans fil à une deuxième sonde à ultrasons filaire, la reprise de l'exploitation de l'ensemble de branchement de sonde (160) pour activer la deuxième sonde à ultrasons filaire, et
la commande du formateur de faisceau (150) de façon qu'il émette le signal de formation de faisceau à destination de la deuxième sonde à ultrasons filaire.

11. Support d'enregistrement lisible par ordinateur sur lequel est enregistré un programme permettant d'exécuter le procédé selon la revendication 8 sur un ordinateur.
